(19) Europäisches Patentamt
European Patent Office
Office européen des brevets

(11) **EP 3 439 529 B1**

(12) **FASCICULE DE BREVET EUROPEEN**

(45) Date de publication et mention
de la délivrance du brevet:
**08.06.2022 Bulletin 2022/23**

(51) Classification Internationale des Brevets (IPC):
***A61B 1/00*** *(2006.01)* ***G02B 23/24*** *(2006.01)*
***G02B 27/00*** *(2006.01)*

(21) Numéro de dépôt: **17719502.1**

(22) Date de dépôt: **04.04.2017**

(52) Classification Coopérative des Brevets (CPC):
**G02B 23/2423; A61B 1/00167; A61B 1/0017;
A61B 1/00172; A61B 1/07; G02B 27/0087**

(86) Numéro de dépôt international:
**PCT/EP2017/058017**

(87) Numéro de publication internationale:
**WO 2017/174596 (12.10.2017 Gazette 2017/41)**

(54) **DISPOSITIFS ET MÉTHODES DE TRANSPORT ET DE CONTRÔLE DE FAISCEAUX LUMINEUX POUR L'IMAGERIE ENDO-MICROSCOPIQUE SANS LENTILLE**

VORRICHTUNGEN UND VERFAHREN ZUR WEITERLEITUNG UND STEUERUNG VON LICHTSTRAHLEN FÜR LINSENLOSE ENDOMIKROSKOPISCHE BILDGEBUNG

DEVICES AND METHODS FOR CONVEYING AND CONTROLLING LIGHT BEAMS FOR LENSLESS ENDO-MICROSCOPIC IMAGERY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.04.2016 FR 1652937**

(43) Date de publication de la demande:
**13.02.2019 Bulletin 2019/07**

(73) Titulaires:
• **Centre National de la Recherche Scientifique CNRS**
**75794 Paris Cedex 16 (FR)**
• **Universite d'Aix-Marseille (AMU)**
**13284 Marseille Cedex 07 (FR)**
• **Ecole Centrale De Marseille (ECM)**
**13013 Marseille (FR)**

(72) Inventeurs:
• **RIGNEAULT, Hervé**
**13190 Allauch (FR)**
• **ANDRESEN, Esben Ravn**
**59800 Lille (FR)**
• **SIVANKUTTY, Siddarth**
**13001 Marseille (FR)**

(74) Mandataire: **Osha Liang**
**2, rue de la Paix**
**75002 Paris (FR)**

(56) Documents cités:
**CN-U- 204 405 899     US-A1- 2011 137 126
US-A1- 2016 022 119**

## Description

## Domaine technique de l'invention

La présente invention concerne des dispositifs et méthodes de transport et de contrôle de faisceaux lumineux pour l'imagerie endo-microscopique dite « sans lentille » ainsi que des systèmes et procédés d'imagerie endo-microscopiques sans lentille. Elle s'applique notamment à l'exploration endoscopique des organes chez un être vivant, humain ou animal.

## Etat de l'art

Les développements en imagerie endo-microscopique nécessitent l'utilisation de dispositifs optomécaniques fibrés présentant des spécificités par rapport aux systèmes imageurs en espace libre.

En effet, la construction d'un microscope miniature qui comprendrait une source lumineuse, une optique de focalisation et une caméra à l'extrémité distale (c'est-à-dire situé en bout de fibre, du côté de l'échantillon) d'un endoscope médical n'est pas envisageable du fait de l'encombrement de l'ensemble des composants. On cherche de ce fait des solutions permettant de réaliser une imagerie en bout de fibre optique tout en limitant l'encombrement à l'extrémité distale de l'endoscope.

Il existe plusieurs approches permettant de réaliser une imagerie en bout de fibre optique tout en limitant l'encombrement à l'extrémité distale de l'endoscope.

Il est connu notamment une technologie qualifiée d' « endoscopie sans lentille », et décrite par exemple dans Cizmar et al. « Exploiting multimode waveguides for pure fibre-based imaging », Nat. Commun. 3, 1027 (2012). Cette technique est basée sur l'utilisation d'une fibre optique multimodes, ou MMF selon l'abréviation de l'expression anglo-saxonne « Multi-Mode Fiber ». La fibre optique MMF est éclairée avec une source cohérente. Du côté proximal (c'est-à-dire en entrée de fibre optique, du côté opposé à l'échantillon) de la fibre optique MMF, un modulateur spatial de front d'onde (SLM pour « Spatial Light Modulator ») permet de jouer sur les modes de propagation de la fibre de telle sorte que l'addition cohérente de ces modes permet de générer la figure d'intensité recherchée en bout de fibre MMF. Dans un mode de réalisation, on cherche par exemple à produire un point focal en bout de fibre MMF et à balayer l'échantillon pour obtenir une image comme on le ferait dans un montage classique de microscopie confocale.

Cette technique, extrêmement puissante du fait du caractère déterministe de la matrice de transmission de la fibre qui relie un champ entrant en partie proximale de la fibre avec un champ sortant en partie distale (et vice versa), permet de s'affranchir de toute optique du côté distal de la fibre multimodes et de ce fait de réduire l'encombrement.

Cependant, la matrice de transmission de la fibre est fortement dépendante de la courbure de la fibre optique MMF. L'imagerie endo-microscopique au moyen d'une fibre optique MMF est donc extrêmement sensible à tout mouvement de la fibre. Par ailleurs, du fait du caractère multimode, une impulsion courte en partie proximale est allongée en partie distale, ce qui limite les possibilités d'application à l'imagerie non linéaire qui nécessite de travailler avec des impulsions lumineuses de fortes intensités crêtes.

En parallèle des technologies basées sur l'utilisation de fibres multimodes, une technologie également de type « sans lentille » s'est développée, basée sur l'utilisation d'un paquet de fibres optiques monomodes (voir par exemple French et al. brevet US 8,585,587). Selon la technique décrite, un modulateur spatial de front d'onde (SLM) agencé du côté proximal du paquet de fibres optiques monomodes permet de contrôler à l'extrémité distale du paquet de fibres le front d'onde émis par une source lumineuse. Cette technique est beaucoup moins sensible aux mouvements, et notamment aux torsions du paquet de fibres optiques. En effet, les modes des fibres optiques monomodes étant localisés et confinés en des points définis sur la surface transverse du paquet de fibres optiques, une torsion du paquet de fibres optiques résulte en une simple translation de la figure d'intensité. Par ailleurs, le caractère monomode des fibres élimine toute dispersion intermodale ; ainsi, la seule contribution à la dispersion est la dispersion chromatique qui est la même pour toutes les fibres optiques monomodes et qui peut donc être compensée de manière globale. De ce fait, l'utilisation d'un paquet de fibres optiques monomodes permet, par rapport aux fibres multimodes, la propagation d'impulsions courtes.

Diverses publications ont décrit des variantes d'endo-microscopie sans lentille basées sur l'utilisation d'un paquet de fibres optiques monomodes et plus précisément d'une fibre multicœurs ou « MCF » selon l'expression anglo-saxonne « Multi-Core Fiber ». Ainsi par exemple, il est montré comment on peut accéder, en partie distale du paquet de fibres optiques, à un balayage très rapide du point de focalisation, en imprimant au moyen d'un dispositif galvanométrique un angle variable du front d'onde en entrée du SLM (voir par exemple E.R. Andresen et al. « Toward endoscopes with no distal optics: video-rate scanning microscopy through a fiber bundle », Opt. Lett. Vol. 38, N°5, 609-611 (2013)). Dans E.R. Andresen et al. (« Two-photon lensless endoscope», Opt. Express 21, N°18 , 20713-20721 (2013)), les auteurs ont démontré la faisabilité expérimentale d'un système d'imagerie non linéaire bi-photonique (TPEF) en endo-microscopie sans lentille. Dans E.R. Andresen et al. (« Measurement and compensation of residual group delay in a multi-core fiber for lensless endoscopy", JOSA B, Vol. 32, No. 6, 1221 - 1228 (2015)), il est décrit un dispositif de contrôle des retards de vitesse de groupe (ou « GDC » pour « Group Delay Control ») pour le transport et le contrôle d'impulsions lumineuses dans un système d'imagerie endo-microscopique sans lentille basé sur l'utilisation d'un paquet de fibres optiques monomodes. Les brevets CN204 405 899 et US 2016/02219 décrivent respectivement un connecteur de fibre multi-cœur et un endoscope à fibre multicœur.

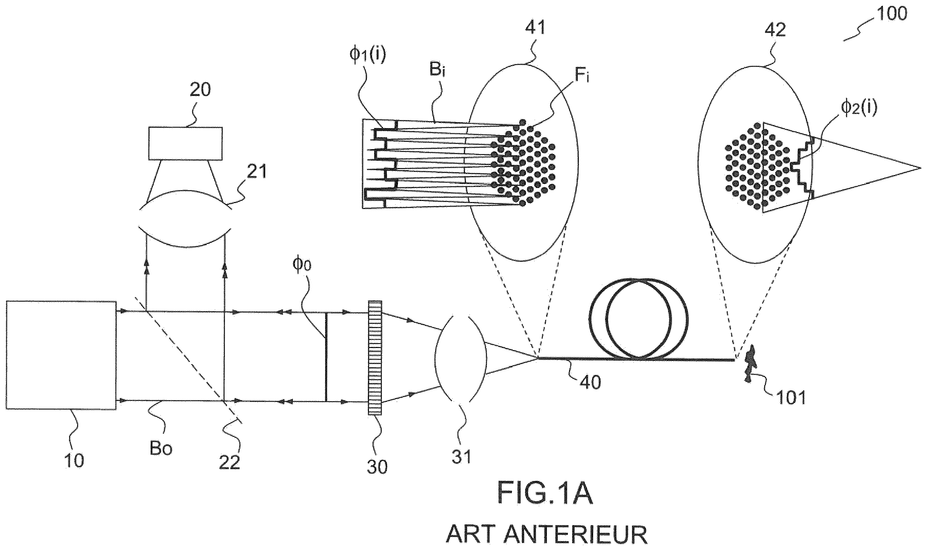

La FIG. 1A illustre de façon schématique un système d'imagerie endo-microscopique sans lentille 100 utilisant un paquet de fibres optiques monomodes. Le système d'imagerie 100 comprend généralement une source d'émission 10 pour l'émission d'un faisceau lumineux incident $B_0$, continu ou formé d'impulsions dans le cas de l'application à l'imagerie non linéaire. Le système 100 comprend par ailleurs une voie de détection comprenant un objectif 21 et un détecteur 20. La voie de détection est séparée de la voie d'émission par une lame séparatrice 22. Le système d'imagerie 100 comprend également un dispositif de transport et de contrôle des faisceaux lumineux permettant d'éclairer un objet d'analyse 101 déporté. Le dispositif de transport et de contrôle comprend un paquet de fibres optiques monomodes 40 dont les faces d'entrée (ou face proximale) 41 et de sortie (ou face distale) 42 sont représentées de façon agrandie sur la figure 1A, et un modulateur spatial de front d'onde (« SLM ») 30 agencé à l'extrémité proximale du paquet de fibres 40 et permettant de contrôler le front d'onde du faisceau émis par la source 10. Le modulateur spatial de lumière permet d'imprimer sur le front d'onde entrant, présentant une fonction de phase $\Phi_0$, un déphasage donné $\Phi_1(i)$ pour chaque faisceau élémentaire $B_i$ destiné à entrer dans une fibre optique $F_i$ du paquet de fibres 40. La fonction de phase $\Phi_1(i)$ pourra être telle que, par exemple, après propagation dans le paquet de fibres optiques, l'onde sorte avec une phase parabolique $\Phi_2(i)$. Cette phase parabolique permet au faisceau de se focaliser du côté distal sur l'objet d'analyse 101 alors qu'il n'y a aucune lentille physique présente ; c'est l'origine de la terminologie « endoscope sans lentille ». Par ailleurs, il est possible grâce au modulateur spatial de lumière de compenser des déphasages introduits par chacune des fibres optiques $F_i$.

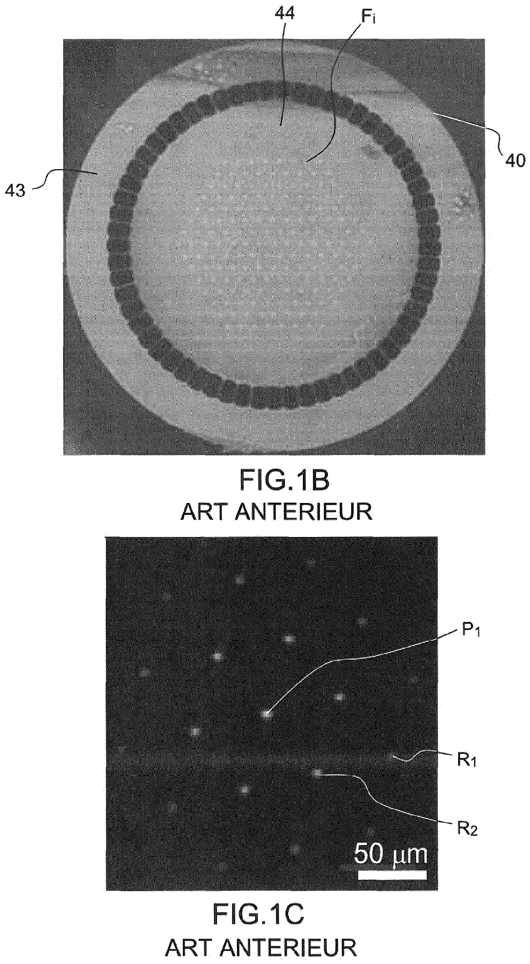

Le paquet de N fibres optiques monomodes peut être formé d'un ensemble de fibres optiques monomodes individuelles, comportant chacune un cœur et une gaine, typiquement une centaine à quelques dizaines de milliers de fibres, rassemblées sous forme d'un faisceau de fibres ; le paquet de N fibres optiques monomodes peut également être formé d'un ensemble de cœurs monomodes d'une fibre multi-cœurs, de préférence au moins une centaine de cœurs monomodes, comme cela est illustré sur la figure 1B. Ainsi, dans l'exemple de la FIG. 1B, une fibre multi-cœurs 40 est représentée qui comprend un ensemble de cœurs monomodes $F_i$, une gaine externe 43 et également dans cet exemple une gaine interne multimodes 44 adaptée pour collecter le signal lumineux rétrodiffusé par l'objet d'analyse depuis l'extrémité distale jusqu'à l'extrémité proximale.

Le plus souvent, pour des questions de procédé de fabrication du paquet de fibres optiques, que ce soit dans le cas d'un paquet formé d'un ensemble de fibres monomodes individuelles ou d'un ensemble de cœurs monomodes d'une fibre multi-cœurs, les fibres monomodes sont agencés de façon périodique ou quasi-périodique au sein du paquet de fibres.

Dans le cas d'un agencement périodique ou quasi-périodique des cœurs, les déposants ont montré qu'une onde avec une phase parabolique $\Phi_2(i)$ résultera dans un plan image donné, non pas en un point image unique mais plutôt, comme cela est montré que la FIG. 1C, en un point central lumineux brillant ($P_1$) entouré de points lumineux ($R_i$) de plus faible intensité, appelés « répliques ». Cet effet résulte directement de la périodicité ou quasi-périodicité de l'agencement des fibres optiques dans le paquet de fibres qui se comporte comme un réseau de diffraction, les répliques résultant de la diffraction à des ordres supérieurs de l'onde se propageant dans le paquet de fibres optiques.

La présente invention propose des dispositifs et méthodes de transport et de contrôle de faisceaux lumineux pour systèmes d'imagerie endo-microscopiques dit « sans lentille » qui permettent de s'affranchir des répliques dans le plan image, quel que soit l'arrangement des fibres optiques au sein du paquet de fibres optiques.

RESUME DE L'INVENTION

Selon un premier aspect, la présente description concerne un dispositif de transport et de contrôle de faisceaux lumineux pour l'imagerie endo-microscopique « sans lentille », c'est-à-dire sans lentille du coté distal, comprenant:

-   un premier guide de lumière comprenant un paquet de fibres optiques monomodes, chaque fibre optique monomode étant destinée à recevoir un faisceau lumineux élémentaire à une extrémité proximale et à émettre un faisceau lumineux à une extrémité distale,

-   un deuxième guide de lumière comprenant un tronçon de fibre optique multimodes, agencé à l'extrémité distale du premier guide de lumière, le tronçon de fibre optique multimodes étant destiné à recevoir les faisceaux lumineux émis par les fibres optiques monomodes du paquet de fibres optiques monomodes;

-   un dispositif optique de contrôle de la phase agencé du côté de l'extrémité proximale du premier guide de lumière comprenant:

    - au moins un premier modulateur spatial de lumière adapté pour l'application d'un déphasage sur chacun des faisceaux élémentaires;
    - des moyens de contrôle du premier modulateur spatial de lumière permettant l'application d'un déphasage sur chacun des faisceaux élémentaires pour former à l'extrémité distale du tronçon de fibre optique multimodes un faisceau d'illumination avec une fonction de phase déterminée.

Les déposants ont montré qu'un dispositif de transport et de contrôle de faisceaux lumineux ainsi décrit permettait le transport de faisceaux lumineux sur de longues distances, typiquement supérieures à 100 cm, sans conséquence du fait d'une éventuelle torsion du paquet de fibres optiques, et avec suppression des répliques. Cet effet est obtenu grâce à l'agencement à l'extrémité distale du paquet de fibres optiques monomodes d'un tronçon de fibre optique multimodes.

Selon un ou plusieurs exemples de réalisation, le tronçon de fibre optique multimodes présente une longueur comprise entre 0,1 mm et 20 mm, avantageusement entre 0,1 et 10 mm. Le tronçon de fibre optique multimodes est alors suffisamment court pour être ultra rigide et suffisamment long pour permettre un brouillage des ordres diffractifs supérieurs en sortie du paquet de fibres optiques monomodes.

Selon l'invention, la fibre optique multimodes est une fibre à saut d'indice, qui permet un brouillage des ordres diffractifs supérieurs en sortie du paquet de fibres optiques monomodes sur un tronçon de longueur très courte, compris par exemple entre 0,1 mm et 5 mm.

Selon un ou plusieurs exemples non pas selon l'invention, la fibre optique multimodes est une fibre à gradient d'indice qui nécessite une longueur plus grande pour obtenir un brouillage des modes, typiquement supérieure à 5 mm.

Selon un ou plusieurs exemples de réalisation, le premier guide de lumière comprend un paquet de N fibres optiques monomodes formé d'un ensemble de fibres optiques monomodes individuelles, comprenant chacune un cœur et une gaine, typiquement une centaine à quelques dizaines de milliers de fibres, rassemblées sous forme d'un faisceau de fibres.

Selon un ou plusieurs exemples de réalisation, le premier guide de lumière comprend un paquet de N fibres optiques monomodes formé d'un ensemble de cœurs monomodes, de préférence au moins une centaine. Par exemple, le premier guide de lumière est une fibre multicœurs et le paquet de N fibres optiques monomodes est formé par les cœurs monomodes de la fibre multi-cœurs.

Selon un ou plusieurs exemples de réalisation, le premier guide de lumière est une fibre multi-cœur à double gaine ; une telle fibre multi-cœur présente l'avantage de transporter avec une grande efficacité le signal lumineux rétrodiffusé dans une gaine de la fibre multi-cœur double gaine, généralement une gaine multimodes.

Par fibre optique monomode, on comprend une fibre dans laquelle la lumière ne peut se propager que dans un seul mode du champ électromagnétique ; par extension on comprend aussi une fibre dite 'monomode effective' qui comprend plusieurs modes mais dans laquelle les conditions de couplage n'excitent qu'un seul mode (généralement le mode fondamental) qui confine la lumière durant toute la propagation (pas de fuite vers les autres modes).

Dans l'ensemble de la description, on pourra utiliser le terme « fibre optique monomode » pour évoquer aussi bien une fibre optique monomode individuelle qu'un cœur monomode d'une fibre multi-cœur.

Selon un ou plusieurs exemples de réalisation, le couplage entre les fibres optiques monomodes du paquet de fibres optiques monomodes est inférieur à -20dB/m, ce qui permet le transport et le contrôle des faisceaux optiques sur une grande longueur du paquet de fibres, tout en offrant la possibilité de compenser les effets de déphasage inter-cœur.

Selon un ou plusieurs exemples de réalisation, le premier modulateur spatial de lumière comprend un miroir déformable segmenté ou à membrane, pour un fonctionnement en réflexion.

Selon un ou plusieurs exemples de réalisation, le premier modulateur spatial de lumière comprend une matrice de cristaux liquides, pour un fonctionnement en réflexion ou en transmission.

Selon un ou plusieurs exemples de réalisation, le dispositif de transport et de contrôle de faisceaux lumineux comprend en outre un système optique adapté au transport des faisceaux lumineux émis par les fibres optiques monomodes du paquet de fibres optiques vers le tronçon de fibre optique multimodes.

Selon un ou plusieurs exemples de réalisation, le système optique permet une conjugaison optique entre une face de sortie du paquet de fibres optiques monomodes et une face d'entrée du tronçon de fibre optique multimodes.

Selon un ou plusieurs exemples de réalisation, une face de sortie du paquet de fibres optiques monomodes et une face d'entrée du tronçon de fibre optique multimodes sont sensiblement confondus avec deux plans focaux dudit système optique.

Selon un ou plusieurs exemples de réalisation, une face de sortie du paquet de fibres optiques monomodes et une face d'entrée du tronçon de fibre optique multimodes sont soudés par fusion et forme une épissure mécanique.

Selon un ou plusieurs exemples de réalisation, le premier guide de lumière comprend une fibre multi-cœur étirée à une extrémité distale pour former une section conique à diamètre dégressif dans laquelle les cœurs monomodes fusionnent pour former le tronçon de fibre multimodes du deuxième guide de lumière. Dans ce cas, la section conique forme la transition entre le paquet de fibre monomodes et le tronçon de fibre multimodes.

Selon un ou plusieurs exemples de réalisation, le dispositif de transport et de contrôle de faisceaux lumineux est adapté au transport et au contrôle de faisceaux lumineux comprenant des impulsions optiques et comprend en outre un dispositif de contrôle des retards de vitesse de groupe des impulsions lumineuses dans le paquet de fibres optiques monomodes.

Selon un deuxième aspect, la présente description concerne un système d'imagerie endo-microscopique comprenant une source lumineuse; un dispositif selon le premier aspect pour le transport et le contrôle des faisceaux lumineux émis par ladite source afin de former un faisceau d'illumination d'un objet avec une fonction de phase déterminée; et une voie de détection destinée à la détection de la lumière renvoyée par l'objet et transmise à travers le deuxième guide de lumière puis à travers le premier guide de lumière, de leur extrémité distale à leur extrémité proximale.

Selon un troisième aspect, la présente description concerne un procédé de transport et de contrôle de faisceaux lumineux pour l'imagerie endo-microscopique sans lentille du coté distal comprenant

- la réception de faisceaux lumineux élémentaire à une extrémité proximale d'un paquet de N fibres optiques monomodes d'un premier guide de lumière, chaque fibre optique monomode étant destinée à recevoir un faisceau lumineux élémentaire et à émettre un faisceau lumineux à une extrémité distale,
- la réception des faisceaux lumineux émis par l'ensemble des fibres optiques monomodes du paquet de fibres optiques par un cœur multimodes d'un tronçon d'une fibre optique multimodes d'un deuxième guide de lumière, agencé à l'extrémité distale du premier guide de lumière;
- l'application au moyen d'au moins un premier modulateur spatial de lumière agencé du côté de l'extrémité proximale du premier guide de lumière d'un déphasage sur chacun des faisceaux élémentaires, afin de former à l'extrémité distale du tronçon de fibre optique multimodes un faisceau d'illumination avec une fonction de phase déterminée.

Selon un ou plusieurs exemples de réalisation, le procédé comprend en outre une calibration préalable permettant de déterminer le déphasage à appliquer sur chacun des faisceaux élémentaires en fonction de la fonction de phase recherchée pour le faisceau d'illumination.

Selon un ou plusieurs exemples de réalisation, l'étape de calibration préalable comprend la détermination partielle ou totale d'une matrice de transmission de l'ensemble formé du paquet de fibres optiques monomodes et de la fibre multimodes.

Selon un ou plusieurs exemples de réalisation, l'application du déphasage sur chacun des faisceaux élémentaires vise à inscrire à l'extrémité distale du tronçon de fibre optique multimodes une fonction de phase déterminée pour former un faisceau d'illumination convergent à une distance donnée d'une face de sortie du tronçon de fibre optique multimodes, permettant de former un point de focalisation.

Selon un ou plusieurs exemples de réalisation, l'application de déphasages successifs sur chacun des faisceaux élémentaires permet un balayage du point de focalisation dans un plan à ladite distance donnée de la face de sortie du tronçon de fibre optique multimodes et/ou à différentes distances de la face de sortie du tronçon de fibre optique multimodes.

Selon un ou plusieurs exemples de réalisation, les faisceaux lumineux élémentaires comprennent des impulsions lumineuses. Le procédé peut alors comprendre, selon un exemple de réalisation, le contrôle de la vitesse de groupe des impulsions lumineuses dans le paquet de fibres optiques monomodes.

Selon un quatrième aspect, la présente description concerne un procédé d'imagerie endo-microscopique sans lentille du coté distal comprenant :

- l'émission de faisceaux lumineux ;
- le transport et le contrôle des faisceaux lumineux au moyen d'un procédé selon le troisième aspect pour l'illumination d'un objet par le faisceau d'illumination;
- la détection de la lumière renvoyée par l'objet et transmise à travers le deuxième guide de lumière puis à travers le premier guide de lumière, de leur extrémité distale à leur extrémité proximale.

La lumière renvoyée par l'objet peut être de différente nature en fonction de l'application ; par exemple la lumière renvoyée est la lumière rétrodiffusée, ou la lumière émise, par exemple par un mécanisme de fluorescence.

BREVE DESCRIPTION DES DESSINS

D'autres avantages et caractéristiques de l'invention apparaîtront à la lecture de la description, illustrée par les figures suivantes :

FIGS. 1A à 1C (déjà décrites), un schéma de principe d'un endoscope dit « sans lentille » selon l'art antérieur, basé sur l'utilisation d'un paquet de fibres monomodes ; une image d'un exemple de fibre multi-cœurs adaptée pour la mise en œuvre d'un endoscope du type décrit sur la FIG. 1A et une image illustrant la présence de répliques;

FIG. 2, un schéma de principe illustrant un exemple de système d'imagerie endo-microscopique sans lentille selon la présente description ;

FIGS. 3A à 3D, des figures illustrant plusieurs exemples d'agencement du paquet de fibres optiques monomodes et de la fibre optique multimodes dans un dispositif de transport et de contrôle selon la présente description ;

FIGS. 4A à 4C, des schémas illustrant des étapes d'un exemple de procédé de transport et de contrôle de faisceaux lumineux selon la présente description;

FIG. 5, un schéma illustrant un exemple de montage expérimental pour la validation d'un procédé de transport et de contrôle de faisceaux lumineux selon la présente description;

FIGS. 6A à 6C, respectivement un schéma de principe d'une validation expérimentale réalisée au moyen du montage de la FIG.5 et des images obtenues ;

FIG. 7, des images obtenues au moyen du montage expérimental de la FIG. 5.

## DESCRIPTION DETAILLEE

Sur les figures, les éléments identiques sont indiqués par les mêmes références.

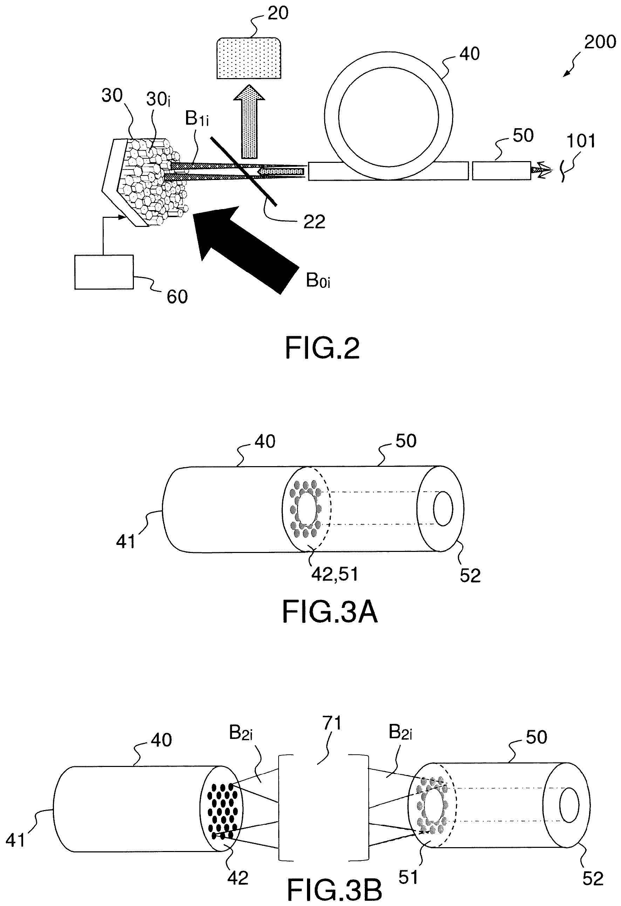

La FIG. 2 illustre de façon schématique un exemple de système 200 d'imagerie endo-microscopique « sans lentille » avec un dispositif de transport et de contrôle de faisceaux lumineux selon la présente description, adapté pour l'imagerie d'un objet référencé 101 sur la FIG. 2.

Le système d'imagerie endo-microscopique 200 comprend une source lumineuse (non représentée sur la FIG. 2) adaptée pour l'émission de faisceaux lumineux $B_{0i}$, les faisceaux lumineux pouvant comprendre des impulsions lumineuses dans le cas d'une application à l'imagerie non linéaire. Ainsi, la source lumineuse comprend par exemple une source laser et, si nécessaire, un système optique de grandissement et de collimation des faisceaux lumineux émis.

Le système d'imagerie endo-microscopique 200 comprend par ailleurs un dispositif pour le transport et le contrôle des faisceaux lumineux émis par ladite source lumineuse afin d'éclairer l'objet 101 selon une figure d'intensité choisie, par exemple un point de focalisation balayé dans le champ, ou d'autres formes, suivant les applications. Le dispositif pour le transport et le contrôle des faisceaux lumineux comprend généralement un premier guide de lumière 40 avec un paquet de fibres optiques monomodes, un deuxième guide de lumière 50 avec un tronçon de fibre optique multimodes, le deuxième guide de lumière 50 étant agencé à une extrémité distale du premier guide de lumière, et un dispositif optique de contrôle de la phase agencé du côté d'une extrémité proximale du premier guide de lumière, comprenant notamment un modulateur spatial de lumière 30.

Le dispositif pour le transport et le contrôle des faisceaux lumineux est dit « sans lentille » car il ne comprend pas de lentille du côté distal, c'est-à-dire du côté de l'émergence des faisceaux lumineux, la phase étant contrôlée par le dispositif de contrôle de la phase agencé du côté d'une extrémité proximale du dispositif.

Dans la suite de la description, on pourra utiliser plus simplement le terme « fibre optique multimodes » pour évoquer le tronçon de fibre optique multimodes. Par ailleurs, le deuxième guide de lumière peut être formé par le tronçon de fibre optique multimodes ou comprendre d'autres éléments, par exemple des éléments de protection, connus de l'homme du métier.

Le paquet de fibres optiques monomodes peut être formé d'un ensemble de fibres optiques monomodes individuelles, typiquement une centaine à quelques dizaines de milliers de fibres, rassemblées sous forme d'un faisceau de fibres, ou peut comprendre un ensemble de cœurs monomodes d'une fibre multi-cœurs, de préférence au moins une centaine.

Ainsi, le premier guide de lumière peut être formé de l'ensemble de fibres optiques monomodes individuelles ou comprendre d'autres éléments, par exemple des éléments de protection, connus de l'homme du métier. Le premier guide de lumière peut également comprendre une fibre multi-cœur à simple gaine ou à double gaine, et comprendre tout autre élément utile à la réalisation du guide, comme des éléments de protection, de façon connue par l'homme du métier. Dans le cas d'une fibre multi-cœurs à double gaine, une gaine peut être une gaine multimodes, adaptée à la propagation du flux lumineux rétrodiffusé par l'objet.

Avantageusement, le couplage entre les fibres optiques monomodes du paquet de fibres optiques monomodes est inférieur à -20dB/m, ce qui permet le transport et le contrôle des faisceaux optiques sur une grande longueur du paquet de fibres, tout en offrant la possibilité de compenser les effets de déphasage inter-cœur.

La longueur des fibres monomodes du paquet de fibres 40 est adaptée à l'application et plus précisément à la longueur requise pour l'endomicroscope. Typiquement, la longueur des fibres monomodes du paquet de fibres est comprise entre 50 cm et 3 m.

Au contraire, le tronçon de fibre optique multimodes est avantageusement choisi le plus court possible, et présente par exemple une longueur comprise entre 0.1 mm et 20 mm, avantageusement entre 0.1 mm et 10 mm. Le tronçon de fibre optique multimodes est alors suffisamment court pour être suffisamment rigide et suffisamment long pour permettre un brouillage de la phase des modes de propagation en sortie du paquet de fibres optiques monomodes.

La fibre optique multimodes peut être par exemple une fibre à gradient d'indice, non pas selon l'invention, ou une fibre à saut d'indice selon l'invention ; dans ce dernier cas, le brouillage de la phase des modes de propagation en sortie du paquet de fibres optiques monomodes peut être obtenu au moyen d'un tronçon de longueur très courte, typiquement compris entre 0.1 mm et 5 mm.

Le tronçon de fibre optique multimodes peut être également destiné à former un implant permanent dans le cas d'applications par exemple à l'imagerie endoscopique du cerveau profond. Dans ce dernier cas, un tronçon de fibre multimodes plus long peut s'avérer intéressant et de ce fait, l'utilisation d'une fibre multimodes à gradient d'indice, peut s'avérer appropriée.

Le dispositif optique de contrôle de la phase est agencé du côté de l'extrémité proximale du paquet de fibres optiques monomodes et comprend le modulateur spatial de lumière 30 adapté pour l'application d'un déphasage sur chacun des faisceaux lumineux élémentaires $B_{0i}$ et une unité de contrôle 60 du modulateur spatial de lumière permettant l'application d'un déphasage sur chacun des faisceaux élémentaires pour l'inscription à l'extrémité distale de la fibre optique multimodes d'une fonction de phase déterminée. Le modulateur spatial de lumière 30 peut comprendre par exemple un miroir déformable segmenté ou à membrane, pour un fonctionnement en réflexion, ou une matrice de cristaux liquides, pour un fonctionnement en réflexion ou en transmission.

Selon un exemple de réalisation, le système d'imagerie 200 peut comprendre aussi des moyens (non représentés sur la FTG. 2) de focalisation des faisceaux lumineux élémentaires $B_{0i}$ sur des éléments 30i du modulateur spatial de lumière 30. Les moyens de focalisation des faisceaux lumineux élémentaires $B_{0i}$ comprennent par exemple une matrice de microlentilles ou un modulateur spatial de lumière, par exemple une matrice de cristaux liquides formant un pavage bidimensionnel de réseaux ayant des phases quadratiques, simulant ainsi une matrice de microlentilles.

Le système d'imagerie endo-microscopique 200 comprend également une voie de détection de la lumière rétrodiffusée par l'objet 101 et transmise à travers la fibre multimodes et le paquet de fibres optiques monomodes de leur extrémité distale à leur extrémité proximale. Sur l'exemple de la FIG. 2, la voie de détection comprend une lame séparatrice de faisceaux 22, un détecteur 20 et éventuellement un objectif (non représenté sur la FIG. 2) pour focaliser la lumière rétrodiffusée sur une surface de détection du détecteur 20, ainsi qu'une unité de traitement (non représenté) des signaux issus du détecteur 20.

Les FIGS. 3A à 3D illustrent plusieurs exemples d'agencement du paquet de fibres optiques monomodes et de la fibre optique multimodes, permettant de faciliter le transport des faisceaux lumineux émis par le paquet de fibres optiques monomodes vers le cœur multimodes de la fibre optique multimodes.

Dans l'exemple de la FIG. 3A, la face de sortie 42 (ou face distale) du paquet de fibres optiques monomodes 40 est maintenue en contact avec une face d'entrée 51 (ou face proximale) de la fibre optique multimodes 50. Il s'agit par exemple d'une soudure directe entre le paquet de fibres optiques monomodes 40 et la fibre optique multimodes 50, ou de n'importe quelle technique de collage optique assurant une bonne transmission lumineuse entre les fibres optiques monomodes et la fibre multimodes.

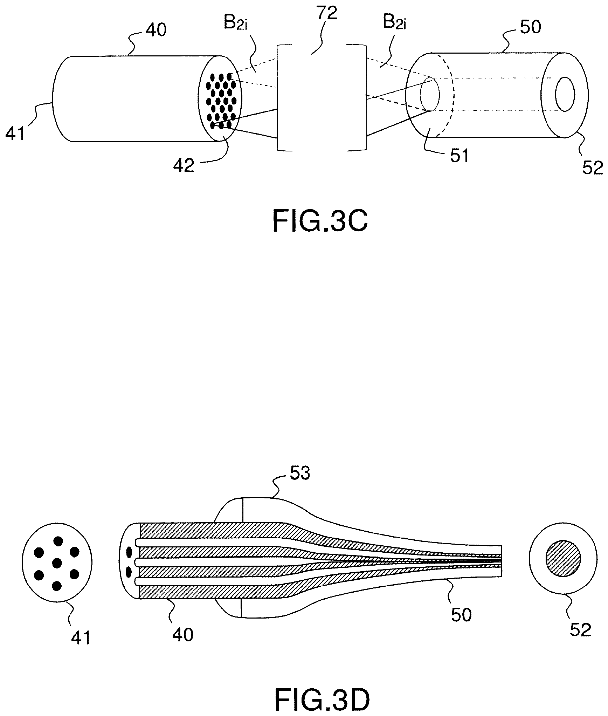

Dans les exemples des FIG. 3B et 3C, un système optique, respectivement référencé 71 et 72 sur chacune des figures, est agencé entre le paquet de fibres optiques monomodes 40 et la fibre optique multimodes pour le transport des faisceaux lumineux émis par les fibres optiques monomodes du paquet de fibres optiques vers le cœur multimodes du tronçon de fibre optique multimodes 50.

Par exemple, comme cela est illustré sur la FIG. 3B, le système optique 71 permet une conjugaison optique entre la face de sortie 42 du paquet de fibres optiques monomodes et la face d'entrée 51 du tronçon de fibre optique multimodes.

Selon un autre exemple, comme cela est illustré sur la FIG. 3C, la face de sortie 42 du paquet de fibres optiques monomodes et la face d'entrée 51 du tronçon de fibre optique multimodes sont sensiblement confondus avec deux plans focaux du système optique 72.

Dans ces deux cas, le réglage du système optique 71 ou du système optique 72 ne nécessite pas d'être parfait, le but étant de faciliter le transport des faisceaux lumineux émis par les fibres optiques monomodes du paquet de fibres optiques vers le cœur multimodes du tronçon de fibre optique multimodes.

La FIG. 3D montre un exemple particulier dans lequel le premier guide de lumière comprend une fibre multi-cœur 40 étirée (ou « tapered » selon l'expression anglo-saxonne) à une extrémité distale pour former une section conique à diamètre dégressif. Dans cette section conique où les cœurs monomodes fusionnent, la propagation devient multimodes comme cela a été décrit par exemple dans l'article de T. A. Birks et al. (« The photonic lantern », Advances in Optics and Photonics 7, 107-167 (2015)); on obtient ainsi le tronçon de fibre multimodes 50 du deuxième guide de lumière. Dans ce cas, la section conique forme la transition entre le paquet de fibre monomodes 40 et le tronçon de fibre multimodes 50. L'ensemble présente une gaine commune 53.

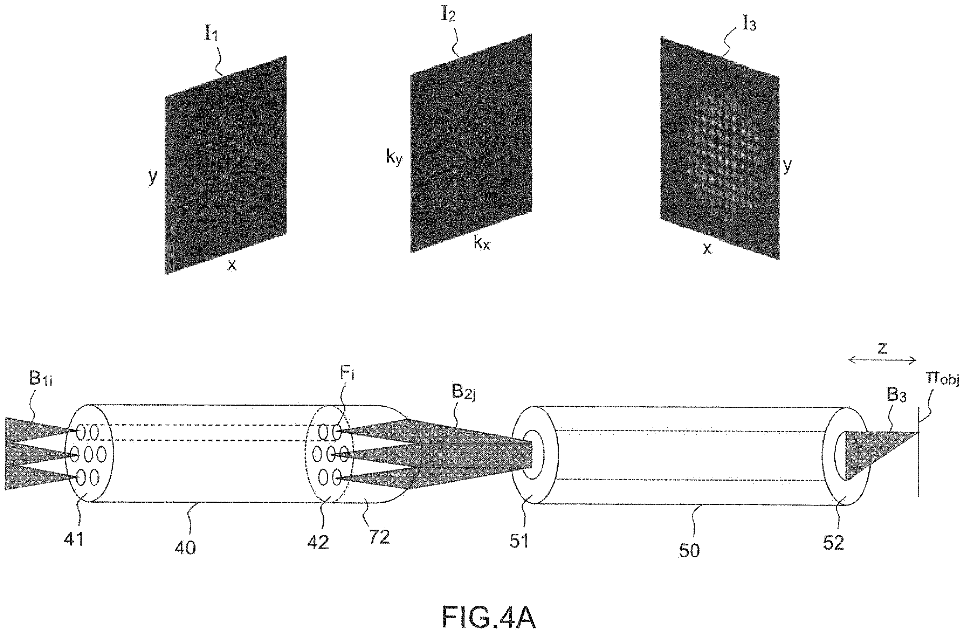

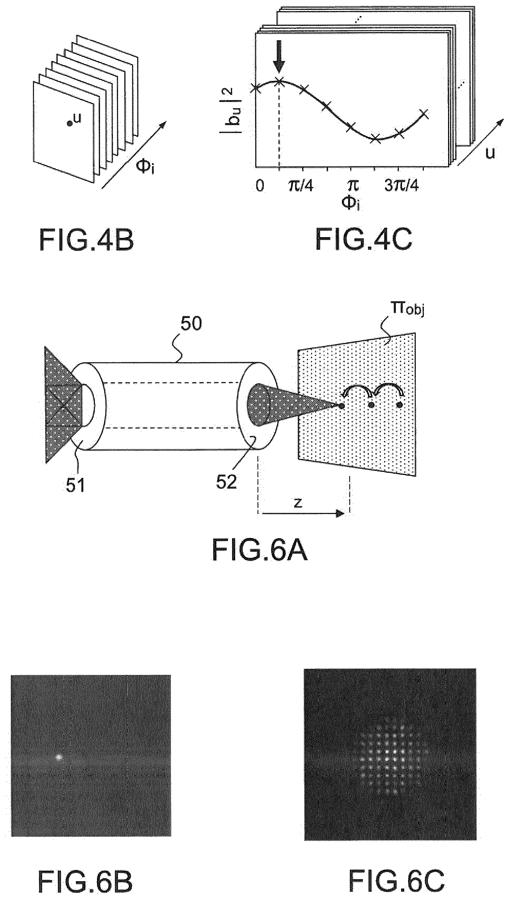

La FIG. 4A illustre de façon plus détaillée des étapes d'un exemple de procédé de transport et de contrôle des faisceaux lumineux selon la présente description, et les FIGS. 4B et 4C des étapes d'un exemple de calibration préalable.

Dans l'exemple choisi pour illustrer le procédé de transport et de contrôle des faisceaux lumineux, un système optique 72, par exemple une lentille, permet le transport des faisceaux lumineux émis par les fibres optiques monomodes du paquet de fibres optiques 40 vers le tronçon de fibre optique multimodes 50, tel qu'illustré par exemple sur la FIG. 3C. Dans cet exemple, la face de sortie 42 du paquet de fibres optiques monomodes et la face d'entrée 51 du tronçon de fibre optique multimodes sont sensiblement confondus avec deux plans focaux ou « plans de Fourier » du système optique 72.

Comme illustré sur la FIG. 4A, des faisceaux lumineux élémentaires $B_{1i}$ dont la phase peut être contrôlée au moyen du modulateur spatial de lumière 30 (non représenté sur la FIG. 4A) sont envoyés sur chacune des fibres monomodes $F_i$ du paquet de fibres 40. Chaque fibre monomode $F_i$ émet alors un faisceau lumineux $B_{2i}$ résultant de la transmission du faisceau lumineux $B_{1i}$ à travers la fibre monomode $F_i$. L'ensemble des faisceaux lumineux $B_{2i}$ sont repérés sur la face de sortie 42 du paquet de fibres optiques monomodes 40 dans l'espace réel (x,y) pour former un ensemble N de modes d'entrée discrets i, associés chacun à une fibre $F_i$, et dont les intensités sont illustrées sur l'image $I_1$ de la FIG. 4A. Du fait que la face de sortie 42 du paquet de fibres optiques monomodes et la face d'entrée 51 du tronçon de fibre optique multimodes sont dans des plans de Fourier du système optique 72, à chaque mode d'entrée i repéré par ses coordonnées réelles $(x_i, y_i)$ correspond un mode intermédiaire j repéré par une direction d'entrée $(kx_j, ky_j)$ dans le plan de la face d'entrée de la fibre multimodes 50. Les intensités des N modes intermédiaires j sont illustrées sur l'image $I_2$ de la FIG. 4A. La fibre multimodes est caractérisée par un nombre fini de modes propres, autrement appelés « eigenmodes ». En pratique, N superpositions (ou combinaisons linéaires) de modes propres sont adressées, N correspondant au nombre de fibres monomodes indexées par i; chaque superposition étant constituée d'un sous-ensemble des modes propres de la fibre multimodes.

On appelle mode de sortie *u* la répartition du champ électromagnétique en sortie de la fibre multimodes 50. On cherche au moyen du procédé selon la présente description à former en sortie du cœur multimodes du tronçon de fibre multimodes 50 le mode de sortie u permettant de former le faisceau d'illumination présentant la fonction de phase et/ou la fonction d'intensité associée recherchée.

Par exemple, comme cela est illustré sur la FIG. 4A, la fonction de phase est déterminée pour former un faisceau $B_3$ convergent pour former un point de focalisation dans un plan situé à une distance z de la face de sortie 52 du tronçon de fibre multimodes 50. L'image $I_3$ sur la FIG. 4A illustre les intensités de modes de sortie u correspondant à différents points de focalisation qui pourront être adressés successivement pendant un balayage.

D'autres formes de faisceaux d'illumination peuvent être recherchées en fonction de l'application. Dans le cas de l'imagerie du cerveau par exemple, on pourra rechercher un faisceau d'illumination dont la forme correspond à celle des éléments (neurones) que l'on veut imager.

La connaissance des déphasages à appliquer sur les faisceaux lumineux élémentaires $B_{1i}$ résulte d'une caractérisation préalable du paquet de fibres optiques monomodes et du tronçon de fibre multimodes.

Par exemple, il est possible de déterminer de façon expérimentale une matrice de transmission complexe totale ou partielle de l'ensemble formé du paquet de fibres optiques monomodes et du tronçon de fibre multimodes. Une matrice de transmission complexe d'un système optique permet d'exprimer généralement l'amplitude et la phase du champ lumineux dans un plan donné en sortie du système optique en fonction de l'amplitude et la phase du champ lumineux dans un plan en entrée du système optique. Grâce à la connaissance de la matrice de transmission, il est possible de caractériser le système formé de l'ensemble du paquet de fibres optiques monomodes et du tronçon de fibre multimodes afin de déterminer le déphasage à appliquer sur chacun des faisceaux lumineux élémentaires $B_{1i}$.

Ainsi, on peut définir une matrice de transmission $K_i^u$ complexe, d'amplitude $A_i^u$ et de phase $P_i^u$ :

$$K_i^u = A_i^u e^{i P_i^u}$$

En pratique, la détermination de la matrice $K_i^u$ peut être partielle et se limiter par exemple à déterminer la matrice $P_i^u$ qui contrôle essentiellement la répartition d'intensité dans le plan de l'objet (l'amplitude $A_i^u$ jouant un rôle marginal). Il est également possible de déterminer la matrice $K_i^u$ de façon incomplète mais cela pourra entraîner une moins bonne précision sur la fonction de phase recherchée pour le faisceau d'illumination.

Pour la détermination de la matrice $K_i^u$ de l'ensemble formé du paquet de fibres optiques monomodes et du tronçon de fibre multimodes, il est possible d'utiliser des méthodes interférentielles basées sur des mesures d'interférence entre l'onde lumineuse en sortie de la fibre optique multimodes et une onde de référence. La figure d'interférence est analysée pour des déphasages successifs appliqués sur chacun des faisceaux élémentaires ou, de manière équivalente sur la référence, ce qui permet de déterminer la matrice $K_i^u$. Ce type de méthode est décrit par exemple dans l'article de Cizmar et al. où l'on cherche à déterminer la matrice de transmission d'une fibre optique multimodes (voir 'Shaping the light transmission through a multimode optical fibre: complex transformation analysis and applications in biophotonics' Opt Express 19, 18871-18884 (2011)).

La matrice de transmission $K_i^u$ étant déterminée, elle peut être enregistrée dans l'unité de contrôle 60 du modulateur spatial de lumière 30, si bien qu'une calibration préalable n'est pas nécessaire pour chaque mise en œuvre du procédé d'imagerie. Alternativement, il est possible avant de démarrer une nouvelle imagerie, de procéder à nouveau à une calibration.

Les figures 4B et 4C illustrent une étape de calibration préalable du procédé de transport et de contrôle des faisceaux lumineux selon la présente description, basé sur une caractérisation du système formé par le paquet de fibres optiques monomodes et le tronçon de fibre multimodes, et adaptée spécifiquement à la formation d'un point de focalisation, qu'il soit ou non balayé.

Dans cet exemple, on suppose que l'on cherche à former des points de focalisation en différents points du plan $\Pi_{obj}$ positionné à une distance z de la face de sortie 52 de la fibre optique multimodes, comme cela est représenté sur la FIG. 4A. Chaque point de focalisation correspond à un faisceau convergent $B_3^k$. En pratique,

on cherchera par exemple à balayer un champ objet prédéterminé et donc à faire varier les déphasages appliqués au moyen du modulateur spatial de lumière 30 sur les faisceaux lumineux élémentaires $B_{1i}$ pour obtenir le balayage recherché du champ objet.

Pour la mise en œuvre de la calibration, un détecteur matriciel, par exemple une caméra, est agencée dans le plan de l'objet $\Pi_{obj}$ ou dans un plan conjugué. A chaque pixel de la caméra correspond un « mode de sortie » référencé *u*. le nombre de modes de sortie *u* correspond ainsi dans cet exemple au nombre M de pixels de la caméra. On cherche à déterminer les phases $\Phi_i$ à appliquer aux faisceaux lumineux élémentaires $B_{1i}$ pour rendre maximale l'intensité de chaque mode de sortie u.

Plus précisément, on peut définir une matrice de transmission complexe $K_i^u$ reliant les N modes d'entrée *i* et les M modes de sortie *u* :

$$B_3^{u=1,M} = K_i^u B_1^{i=1,N}$$

le nombre N de modes d'entrée est limité par le nombre de fibres optiques monomodes dans le paquet de fibres 40 et le nombre M de modes de sortie est limité par le nombre de pixels de la caméra.

Comme précédemment expliqué, la matrice de transmission $K_i^u$ complexe, d'amplitude $A_i^u$ et de phase $P_i^u$ :

$$K_i^u = A_i^u e^{i P_i^u}$$

En pratique, la détermination de la matrice $K_i^u$ revient à mesurer seulement la matrice $P_i^u$ qui contrôle essentiellement la répartition d'intensité dans le plan de l'objet.

La détermination de la matrice $P_i^u$ peut comprendre les étapes suivantes :

     Envoi de deux modes d'entrée, un mode de référence *i* = 0 et un mode d'entrée *i* auquel il est adjoint une phase $\Phi_i$;
     Enregistrement pour chaque mode de sortie u, c'est-à-dire pour chaque pixel de la caméra, de l'intensité résultante pour un nombre donné de laveurs équidistantes de $\Phi_i$, par exemple 8, entre 0 et $2\pi$, comme cela est montré sur la FIG. 4C ;
     Enregistrement pour chaque mode de sortie u de la phase $\Phi_i$ qui rend maximale l'intensité (FIG. 4B) ;
     Réitération pour chaque mode d'entrée i.

Bien entendu, les étapes de calibration du procédé de transport et de contrôle des faisceaux lumineux décrits précédemment peuvent s'appliquer également quand le paquet de fibres optiques monomodes 40 et le tronçon de fibre multimodes 50 sont agencés différemment.

Notamment, si la face d'entrée 51 est dans le même plan, ou dans un plan conjugué avec la face de sortie 42 du paquet de fibres monomodes 40, le plan intermédiaire associé à la face d'entrée 51 de la fibre multimodes 50 peut être indexé dans l'espace réel (x, y).

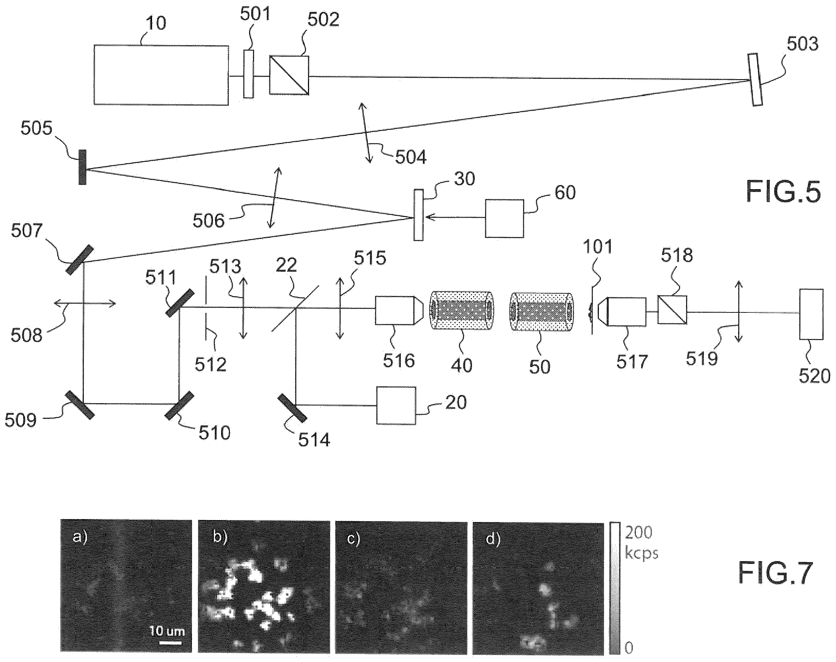

La FIG. 5 représente un exemple de montage expérimental mis en œuvre pour la validation d'un procédé de transport et de contrôle de faisceaux lumineux selon la présente description.

Le montage expérimental comprend une source laser 10 émettant un faisceau lumineux envoyé sur un « façonneur de front d'onde » 503, par exemple une matrice de microlentilles ou un modulateur spatial de lumière à deux dimensions inscrivant un réseau de phases quadratiques simulant une matrices de microlentilles, et permettant de former un ensemble de faisceaux élémentaires focalisés sur les segments d'un miroir déformable segmenté 30. Un télescope 504, 506 permet d'ajuster la dimension du faisceau dans le plan du miroir déformable 30. Chaque segment du miroir déformable 30 est imagé sur une fibre monomode du paquet de fibres optiques monomodes 40 (imageur 508, 513, 515, 516). Un dispositif de contrôle 60 du miroir déformable 30 permet de contrôler la phase $\Phi_i$ associée à chaque mode d'entrée i et correspondant à chacun des faisceaux élémentaires. Une lentille de focale f = 500 $\mu$m (non visible sur la FIG. 5) est placée sur la face de sortie du paquet de fibres 40 de telle sorte que la face de sortie du paquet de fibres 40 et la face d'entrée de la fibre multimodes 50 se trouvent dans des plans de Fourier. La répartition de l'intensité dans le plan de l'objet 101 est observée au moyen d'une caméra CMOS 520 comprenant M = 4096 pixels et conjuguée avec le plan de l'objet 101 au moyen d'in objectif 517. L'ensemble formé d'une lame demi-onde 501 et d'un polariseur 502 permet d'ajuster l'état de polarisation afin qu'il coïncide avec celui pour lequel le façonneur de front d'onde 503 est actif dans le cas ou ce composant utilise des cristaux liquides ; d'autre part ce montage permet d'ajuster la puissance envoyée sur l'échantillon 101. Un polariseur 518 permet de sélectionner un seul état de polarisation pour lequel la matrice de transmission est mesurée et le point de focalisation dans le plan $\Pi_{obj}$ optimisé. Une lame séparatrice 22 permet de renvoyer vers un détecteur 20 la lumière rétrodiffusée ou émise (dans le cas de la fluorescence) par l'objet et transmise du côté distal au côté proximal par le tronçon de fibre multimodes et le premier guide de lumière. Par exemple, le détecteur 20 est un photomultiplicateur ou une photodiode à avalanche. Lorsqu'on balaye l'échantillon 101 à l'aide d'un faisceau focalisé, on collecte le signal rétrodiffusé ou émis par chaque point de l'échantillon à l'aide du détecteur 101 afin de former une image.

Une calibration du procédé de transport et de contrôle des faisceaux lumineux mis en œuvre grâce au montage expérimental de la FIG. 5 a par ailleurs été réalisée afin de contrôler la positon d'un point de focalisation dans le champ de l'objet $\Pi_{obj}$ comme cela est illustré sur le schéma de la FIG. 6A. La calibration est réalisée selon le protocole de détermination de la matrice de transmission décrit précédemment.

Dans l'exemple montré sur la FIG. 6A, le plan de l'objet se trouve à une distance z = 250 $\mu$m de la face de sortie du tronçon de fibre multimodes.

On observe sur la FIG. 6B un point de focalisation dans le plan de l'objet obtenu grâce au dispositif montré sur la FIG. 5. Comme cela est visible, aucune réplique n'est apparente. La FIG. 6C représente un sous-ensemble de points de focalisation correspondant à différents modes de sortie u obtenus par application de phases $\Phi_i$ sur les lumineux élémentaires $B_{1i}$. Dans la pratique un seul point de focalisation n'est visible comme indiqué sur la Fig. 6B, la Fig. 6C montre plusieurs points pour apprécier le champ de vue.

Ainsi, les déposants ont montré tant théoriquement qu'expérimentalement, qu'après une distance de propagation dans le cœur de la fibre multimodes très courte, typiquement 1 mm ou quelques millimètres en fonction de la nature de la fibre, les modes de propagation présentent des phases aléatoires. Cette nature aléatoire des phases associées à chaque mode de propagation de la fibre multimodes est à l'origine même de la disparition des répliques. Les déphasages relatifs entre les modes de la fibre multimodes résultants de la propagation, on comprend pourquoi une fibre multimodes à saut d'indice est plus efficace qu'une fibre multimodes à gradient d'indice pour brouiller les modes ; en effet dans une fibre à saut d'indice, les constantes de propagations associées à chacun des modes sont plus dispersées, donnant lieu à des déphasages différentiels plus importants.

Bien que les phases accumulées par les différents modes, lors de la propagation dans la fibre multimodes, soient finalement aléatoires, elles sont cependant déterministes et sont inclus dans la détermination de la matrice de transmission englobant le premier guide de lumière et la fibre optique multimodes.

Les déposants ont ainsi démontré la faisabilité d'un dispositif de transport et de contrôle de faisceaux lumineux pour l'endo-microscopie sans lentille, une fonction de balayage du champ de l'objet à une distance donnée z de la face de sortie 52 du tronçon de fibre optique multimodes 50 pouvant être obtenue par contrôle des déphasages appliqués au moyen du modulateur spatial de lumière 30.

Le dispositif de transport et de contrôle de faisceaux lumineux selon la présente description permet également de choisir la distance z du plan de l'objet. Pour cela, une calibration telle que décrite précédemment peut être effectuée pour un ensemble de valeurs z de la distance entre le plan de l'objet et la face de sortie 52 du tronçon de fibre optique multimodes.

La FIG. 7 montre ainsi des images de billes fluorescentes obtenues au moyen du montage expérimental de la FIG. 5 et détectées par le détecteur 20, pour des valeurs de z égales à z = 10 $\mu$m (a), z = 40 $\mu$m (b), z = 70 $\mu$m (c), z = 100 $\mu$m (d).

Plus précisément, la source lumineuse utilisée pour obtenir ces images est un laser Titane :Saphir à 800 nm émettant des impulsions de 200 fs ; les images obtenues sont des images à deux photons, le détecteur 20 est une photodiode à avalanche.

Ces résultats expérimentaux démontrent ainsi également l'application du procédé de transport et de contrôle de faisceaux lumineux en imagerie non linéaire, le dispositif étant adapté à la transmission d'impulsions courtes.

Cependant, dans le cas de manipulations d'impulsions ultra-courtes, le dispositif de transport et de contrôle de faisceaux lumineux selon la présente description peut comprendre également un dispositif de contrôle de la vitesse de groupe des impulsions lumineuses dans le paquet de fibres optiques monomodes, comme cela est décrit dans la publication de E.R. Andresen et al. (« Measurement and compensation of residual group delay in a multi-core fiber for lensless endoscopy", JOSA B, Vol. 32, No. 6, 1221 - 1228 (2015)).

Il est ainsi possible grâce au procédé décrit de réaliser de l'imagerie endo-microscopique sans lentille. Outre le transport et le contrôle des faisceaux lumineux au moyen du procédé décrit précédemment, le procédé d'imagerie endo-microscopique pourra comprendre la détection de la lumière rétrodiffusée par l'objet et transmise à travers la fibre multimodes et le paquet de fibres optiques monomodes de leur extrémité distale à leur extrémité proximale.

Bien que décrits à travers un certain nombre d'exemples de réalisation détaillés, le dispositif de transport et de contrôle d'impulsions lumineuses pour l'imagerie endo-microscopique dite « sans lentille » ainsi que les systèmes et procédés d'imagerie endo-microscopiques sans lentille comprennent différentes variantes, modifications et perfectionnements qui apparaîtront de façon évidente à l'homme de l'art, étant entendu que ces différentes variantes, modifications et perfectionnements font partie de la portée de l'invention, telle que définie par les revendications qui suivent.

**Revendications**

1. Dispositif de transport et de contrôle de faisceaux lumineux pour l'imagerie endo-microscopique sans lentille du coté distal comprenant :

   - un premier guide de lumière (40) comprenant un paquet de fibres optiques monomodes ($F_i$), chaque fibre optique monomode ($F_i$) étant configurée pour recevoir un faisceau lumineux élémentaire ($B_{1i}$) à une extrémité proximale et à émettre un faisceau lumineux ($B_{2i}$) à une extrémité distale,
- un deuxième guide de lumière (50) comprenant un tronçon de fibre optique multimodes à saut d'indice, agencé à l'extrémité distale du premier guide de lumière, le tronçon de fibre optique multimodes étant configuré pour recevoir les faisceaux lumineux ($B_{2i}$) émis par l'ensemble des fibres optiques monomodes du paquet de fibres optiques ;
- un dispositif optique de contrôle de la phase agencé du côté de l'extrémité proximale du premier guide de lumière (40) comprenant:

  - au moins un premier modulateur spatial de lumière (30) configuré pour l'application d'un déphasage sur chacun des faisceaux élémentaires ($B_{1i}$) ;
  - des moyens de programmation (60) du premier modulateur spatial de lumière configurés pour l'application d'un déphasage sur chacun des faisceaux élémentaires ($B_{1i}$) pour former à l'extrémité distale de la fibre optique multimodes (50) un faisceau d'illumination avec une fonction de phase déterminée.

2. Dispositif de transport et de contrôle de faisceaux lumineux selon la revendication 1, dans lequel le tronçon de fibre optique multimodes présente une longueur comprise entre 0.1 mm et 20 mm.

3. Dispositif de transport et de contrôle de faisceaux lumineux selon l'une quelconque des revendications précédentes, comprenant en outre un système optique (71, 72) adapté au transport des faisceaux lumineux émis par les fibres optiques monomodes du paquet de fibres optiques vers le tronçon de fibre optique multimodes.

4. Dispositif de transport et de contrôle de faisceaux lumineux selon l'une quelconque des revendications précédentes, dans lequel le premier guide de lumière (40) comprend une fibre multi-cœurs à double gaine.

5. Système d'imagerie endo-microscopique comprenant :

  - une source lumineuse (10) pour l'émission de faisceaux lumineux ;
  - un dispositif selon l'une quelconque des revendications précédentes pour le transport et le contrôle des faisceaux lumineux émis par ladite source pour la formation d'un faisceau d'illumination d'un objet avec une fonction de phase déterminée; et
  - une voie de détection (20, 21) destinée à la détection de la lumière renvoyée par l'objet et transmise à travers le deuxième guide de lumière (50) puis à travers le premier guide de lumière (40), de leur extrémité distale à leur extrémité proximale.

6. Procédé de transport et de contrôle de faisceaux lumineux pour l'imagerie endo-microscopique sans lentille du coté distal comprenant :

  - la réception de faisceaux lumineux élémentaire ($B_{1i}$) à une extrémité proximale d'un paquet de N fibres optiques monomodes ($F_i$) d'un premier guide de lumière (40), chaque fibre optique monomode ($F_i$) étant destinée à recevoir un faisceau lumineux élémentaire ($B_{1i}$) et à émettre un faisceau lumineux ($B_{2i}$) à une extrémité distale,
  - la réception des faisceaux lumineux émis par l'ensemble des fibres optiques monomodes du paquet de fibres optiques par un cœur multimodes d'un tronçon d'une fibre optique multimodes à saut d'indice d'un deuxième guide de lumière (50), agencé à l'extrémité distale du premier guide de lumière (40);
  - l'application au moyen d'au moins un premier modulateur spatial de lumière agencé du côté de l'extrémité proximale du premier guide de lumière d'un déphasage sur chacun des faisceaux élémentaires, afin de former à l'extrémité distale du tronçon de fibre optique multimodes un faisceau d'illumination ($B_3$) avec une fonction de phase déterminée.

7. Procédé selon la revendication 6, comprenant en outre une calibration préalable permettant de déterminer le déphasage à appliquer sur chacun des faisceaux élémentaires ($B_{1i}$) en fonction de la fonction de phase recherchée pour le faisceau d'illumination ($B_3$).

8. Procédé selon l'une quelconque des revendications 6 à 7, dans lequel l'application du déphasage sur chacun des faisceaux élémentaires vise à former à l'extrémité distale du tronçon de fibre optique multimodes (50) un faisceau d'illumination convergent à une distance donnée (z) d'une face de sortie (52) du tronçon de fibre optique multimodes (50) permettant de former un point de focalisation.

9. Procédé selon la revendication 8, dans lequel l'application de déphasages successifs sur chacun des faisceaux élémentaires permet un balayage dudit point de focalisation dans un plan ($\Pi_{obj}$) à ladite distance donnée de la face de sortie du tronçon de fibre optique multimodes et/ou à différentes distances de la face de sortie du tronçon de fibre optique multimodes.

**10.** Procédé d'imagerie endo-microscopique sans lentille du coté distal comprenant :

- l'émission de faisceaux lumineux ;
- le transport et le contrôle des faisceaux lumineux au moyen d'un procédé tel que décrit selon l'une quelconque des revendications 6 à 9 pour l'illumination d'un objet par le faisceau d'illumination ($B_3$);
- la détection de la lumière renvoyée par l'objet et transmise à travers le deuxième guide de lumière (50) puis à travers le premier guide de lumière (40), de leur extrémité distale à leur extrémité proximale.

**Patentansprüche**

**1.** Vorrichtung zum Transport und zur Steuerung von Lichtstrahlen für die auf der distalen Seite linsenlose endomikroskopische Bildgebung, aufweisend:

- einen ersten Lichtleiter (40), der ein Bündel von Singlemode-Lichtleitfasern ($F_i$) aufweist, wobei jede Singlemode-Lichtleitfaser ($F_i$) so konfiguriert ist, dass sie einen elementaren Lichtstrahl ($B_{1i}$) an einem proximalen Ende empfängt und einen Lichtstrahl ($B_{2i}$) an einem distalen Ende aussendet,
- einen zweiten Lichtleiter (50) aufweisend einem Abschnitt einer Multimode-Indexsprung-Lichtleitfaser, der am distalen Ende des ersten Lichtleiters angeordnet ist, wobei der Abschnitt der Multimode-Lichtleitfaser so konfiguriert ist, dass er die Lichtstrahlen ($B_{2i}$) empfängt, die von der Gesamtheit der Singlemode-Lichtleitfasern des Lichtleitfaserpakets ausgesendet werden;
- eine optische Vorrichtung zur Phasensteuerung, die auf der Seite des proximalen Endes des ersten Lichtleiters (40) angeordnet ist, aufweisend:

- mindestens einen ersten räumlichen Lichtmodulator (30), der für die Anwendung einer Phasenverschiebung auf jeden der elementaren Lichtstrahlen ($B_{1i}$) konfiguriert ist;
- Programmiermittel (60) des ersten räumlichen Lichtmodulators, die so konfiguriert sind, dass sie eine Phasenverschiebung auf jeden der elementaren Lichtstrahlen ($B_{1i}$) anwenden, um am distalen Ende der Multimode-Lichtleitfaser (50) einen Beleuchtungsstrahl, aufweisend eine bestimmte Phasenfunktion, zu bilden.

**2.** Vorrichtung zum Transport und zur Steuerung von Lichtstrahlen nach Anspruch 1, wobei der Abschnitt der Multimode-Lichtleitfaser eine Länge zwischen 0,1 mm und 20 mm aufweist.

**3.** Vorrichtung zum Transport und zur Kontrolle von Lichtstrahlen nach einem der vorhergehenden Ansprüche, die außerdem ein optisches System (71, 72) aufweist, das zum Transport der von den Singlemode-Lichtleitfasern des Lichtleitfaserpakets emittierten Lichtstrahlen zum Multimode-Lichtleitfaserabschnitt geeignet ist.

**4.** Vorrichtung zum Transport und zur Steuerung von Lichtstrahlen nach einem der vorhergehenden Ansprüche, wobei der erste Lichtleiter (40) eine doppelt ummantelte Multicore-Faser aufweist.

**5.** System zur endomikroskopischen Bildgebung, aufweisend:

- einer Lichtquelle (10) zum Aussenden von Lichtstrahlen;
- eine Vorrichtung nach einem der vorhergehenden Ansprüche zum Transport und zur Steuerung der von der Quelle emittierten Lichtstrahlen, um einen Beleuchtungsstrahl zur Beleuchtung eines Objekts, aufweisend eine bestimmte Phasenfunktion, zu bilden; und
- einen Detektionspfad (20, 21) zum Erfassen von Licht, das von dem Objekt zurückgeworfen und durch den zweiten Lichtleiter (50) und dann durch den ersten Lichtleiter (40) von ihrem distalen Ende zu ihrem proximalen Ende übertragen wird.

**6.** Verfahren zum Transport und zur Steuerung von Lichtstrahlen für die auf der distalen Seite linsenlose endomikroskopische Bildgebung, aufweisend:

- Empfangen von elementaren Lichtstrahlen ($B_{1i}$) an einem proximalen Ende eines Pakets aus N Singlemode-Lichtleitfasern (Fi) eines ersten Lichtleiters (40), wobei jede Singlemode-Lichtleitfaser ($F_i$) dazu bestimmt ist, einen elementaren Lichtstrahl ($B_{1i}$) zu empfangen und einen Lichtstrahl ($B_{2i}$) an einem distalen Ende auszusenden,
- Empfangen der Lichtstrahlen, die von allen Singlemode-Lichtleitfasern des Lichtleitfaserpakets ausgesendet werden, durch einen Multimode-Kern eines Abschnitts einer Multimode-Indexsprung-Lichtleitfaser eines zweiten Lichtleiters (50), der am distalen Ende des ersten Lichtleiters (40) angeordnet ist;
- Anwenden einer Phasenverschiebung an jeden der Elementarstrahlen mittels mindestens eines ersten räumlichen Lichtmodulators, der an der Seite des proximalen Endes des ersten Lichtleiters angeordnet ist, um am distalen Ende des Abschnitts der Multimode-Lichtleitfaser ei-

nen Beleuchtungsstrahl ($B_3$), aufweisend eine bestimmte Phasenfunktion, zu bilden.

7. Verfahren nach Anspruch 6, das außerdem eine vorherige Kalibrierung aufweist, die es ermöglicht, die auf jeden der elementaren Lichtstrahlen ($B_{1i}$) anzuwendende Phasenverschiebung in Abhängigkeit von der für den Beleuchtungsstrahl ($B_3$) angestrebten Phasenfunktion zu bestimmen.

8. Verfahren nach einem der Ansprüche 6 bis 7, bei dem die Anwendung der Phasenverschiebung auf jeden der elementaren Lichtstrahlen darauf abzielt, am distalen Ende des Multimode-Lichtleitfaserabschnitts (50) einen Beleuchtungsstrahl zu bilden, der in einem gegebenen Abstand (z) von einer Ausgangsfläche (52) des Multimode-Lichtleitfaserabschnitts (50) konvergiert, um einen Fokuspunkt zu bilden.

9. Verfahren nach Anspruch 8, bei dem die Anwendung von aufeinanderfolgenden Phasenverschiebungen auf jeden der elementaren Lichtstrahlen eine Abtastung des genannten Fokuspunkts in einer Ebene ($\Pi_{obj}$) in dem gegebenen Abstand von der Austrittsfläche des Multimode-Lichtwellenleiterabschnitts und/oder in verschiedenen Abständen von der Austrittsfläche des Multimode-Lichtwellenleiterabschnitts ermöglicht.

10. Verfahren zur auf der distalen Seite linsenlose endomikroskopischen Bildgebung, aufweisend:

- Aussenden von Lichtstrahlen;
- Transport und Steuerung der Lichtstrahlen mittels eines Verfahrens, wie es nach einem der Ansprüche 6 bis 9 beschrieben ist, zur Beleuchtung eines Objekts durch den Beleuchtungsstrahl ($B_3$);
- Erfassen von Licht, das von dem Objekt zurückgeworfen und durch den zweiten Lichtleiter (50) und dann durch den ersten Lichtleiter (40) von ihrem distalen Ende zu ihrem proximalen Ende übertragen wird.

**Claims**

1. A device for transporting and controlling light beams for endo-microscopic imaging without a lens on the distal side, comprising:

- a first light guide (40) comprising a bundle of single-mode optical fibres ($F_i$), wherein each single-mode optical fibre ($F_i$) is configured to receive an elementary light beam ($B_{1i}$) at a proximal end and emit a light beam ($B_{2i}$) at a distal end,

- a second light guide (50) comprising a section of a step-index multimode optical fibre, arranged at the distal end of the first light guide, wherein the multimode optical fibre section is configured to receive the light beams ($B_{2i}$) emitted by the single-mode optical fibres of the optical fibre bundle;
- an optical device for phase control arranged on the side of the proximal end of the first light guide (40) comprising:

- at least one spatial light modulator (30) configured to apply a phase shift to each of the elementary beams ($B_{1i}$);
- means of programming (60) the first spatial light modulator configured for the application of a phase shift to each of the elementary beams ($B_{1i}$) in order to form an illumination beam with a determined phase function at the distal end of the multimode optical fibre (50).

2. The device for transporting and controlling light beams according to claim 1, wherein the multimode optical fibre section has a length of between 0.1 mm and 20 mm.

3. The device for transporting and controlling light beams according to any one of the preceding claims, further comprising an optical system (71, 72) adapted to transport of the light beams emitted by the single-mode optical fibres of the optical fibre bundle to the multimode optical fibre section.

4. The device for transporting and controlling light beams according to any one of the preceding claims, wherein the first light guide (40) comprises a double-clad multi-core fibre.

5. An endo-microscopic imaging system comprising:

- a light source (10) for emitting light beams;
- a device according to any one of the preceding claims for transporting and controlling the light beams emitted by said source for forming an illumination beam for illuminating an object with a determined phase function; and
- a detection channel (20, 21) designed to detect the light reflected by the object and transmitted through the second light guide (50) and subsequently through the first light guide (40), from their distal end to their proximal end.

6. A method for transporting and controlling light beams for endo-microscopic imaging without a lens on the distal side, comprising:

- receiving elementary light beams ($B_{1i}$) at a

proximal end of a bundle of N single-mode optical fibres ($F_i$) of a first light guide (40), wherein each single-mode optical fibre ($F_i$) is intended to receive an elementary light beam ($B_{1i}$) and emit a light beam ($B_{2i}$) at a distal end,
- receiving the light beams emitted by all the single-mode optical fibres of the optical fibre bundle via a multimode core of a section of a step-index multimode optical fibre of a second light guide (50), arranged at the distal end of the first light guide (40);
- applying a phase shift to each of the elementary beams, by means of at least one first spatial light modular arranged on the side of the proximal end of the first light guide, in order to form an illumination beam ($B_3$) with a determined phase function at the distal end of the multimode optical fibre section.

7. The method according to claim 6, further comprising a preliminary calibration serving to determine the phase shift to be applied to each of the elementary beams ($B_{1i}$) depending on the phase function sought for the illumination beam ($B_3$).

8. The method according to any one of claims 6 to 7, wherein application of the phase shift to each of the elementary beams aims to form at the distal end of the multimode optical fibre section (50) a convergent illumination beam at a given distance (z) from an output facet (52) of the multimode optical fibre section (50), enabling formation of a focal point.

9. The method according to claim 8, wherein application of successive phase shifts to each of the elementary beams allows scanning of said focal point in a plane ($II_{obj}$) at said given distance from the output facet of the multimode optical fibre section and/or at different distances from the output facet of the multimode optical fibre section.

10. The method for endo-microscopic imaging without a lens on the distal side, comprising:

   - emission of light beams;
   - transport and control of the light beams by means of a method as described according to any one of claims 6 to 9 for illuminating an object by the illumination beam ($B_3$);
   - detection of the light reflected by the object and transmitted through the second light guide (50) and subsequently through the first light guide (40), from their distal end to their proximal end.

FIG.1A
ART ANTERIEUR

FIG.1B
ART ANTERIEUR

FIG.1C
ART ANTERIEUR

FIG.2

FIG.3A

FIG.3B

FIG.3C

FIG.3D

FIG.4A

FIG.4B

FIG.4C

FIG.6A

FIG.6B

FIG.6C

EP 3 439 529 B1

FIG.5

FIG.7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8585587 B, French
- CN 204405899
- US 201602219 B

**Littérature non-brevet citée dans la description**

- **CIZMAR et al.** Exploiting multimode waveguides for pure fibre-based imaging. *Nat. Commun.,* 2012, vol. 3, 1027
- **E.R. ANDRESEN et al.** Toward endoscopes with no distal optics: video-rate scanning microscopy through a fiber bundle. *Opt. Lett.,* 2013, vol. 38 (5), 609-611
- **E.R. ANDRESEN et al.** Two-photon lensless endoscope. *Opt. Express,* 2013, vol. 21 (18), 20713-20721
- **E.R. ANDRESEN et al.** Measurement and compensation of residual group delay in a multi-core fiber for lensless endoscopy. *JOSA B,* 2015, vol. 32 (6), 1221-1228
- **T. A. BIRKS et al.** The photonic lantern. *Advances in Optics and Photonics,* 2015, vol. 7, 107-167
- Shaping the light transmission through a multimode optical fibre: complex transformation analysis and applications in biophotonics. *Opt Express,* 2011, vol. 19, 18871-18884